(19) Europäisches Patentamt
European Patent Office
Office européen des brevets

(11) **EP 0 495 140 B1**

(12) **EUROPÄISCHE PATENTSCHRIFT**

(45) Veröffentlichungstag und Bekanntmachung des Hinweises auf die Patenterteilung:
**18.06.1997 Patentblatt 1997/25**

(51) Int. Cl.$^6$: **A61B 17/39**

(21) Anmeldenummer: **91100442.2**

(22) Anmeldetag: **16.01.1991**

(54) **Hochfrequenz-Chirurgiegerät**

H.F. electrosurgical unit

Appareil pour électrochirurgie à haute fréquence

(84) Benannte Vertragsstaaten:
**DE FR GB IT**

(43) Veröffentlichungstag der Anmeldung:
**22.07.1992 Patentblatt 1992/30**

(73) Patentinhaber: **Erbe Elektromedizin GmbH.**
**D-72072 Tübingen (DE)**

(72) Erfinder:
• **Farin, Günter**
 **W-7400 Tübingen (DE)**
• **Mausberg, Rainer**
 **W-3400 Göttingen (DE)**
• **Visser, Heiko**
 **W-3400 Göttingen (DE)**
• **Fastenmeier, Karl**
 **W-8000 München 83 (DE)**
• **Lohr, Georg**
 **W-8012 Ottobrunn (DE)**

(74) Vertreter: **Endlich, Fritz, Dipl.-Phys.**
**Patentanwalt et al**
**Meissner, Bolte & Partner**
**Postfach 86 06 24**
**81633 München (DE)**

(56) Entgegenhaltungen:
EP-A- 0 341 446          FR-A- 2 417 303
GB-A- 2 213 381

## Beschreibung

Die Erfindung betrifft ein Zahnmedizinisches Hochfrequenz-Chirurgiegerät mit einem Hochfrequenzgenerator zum Schneiden und/oder Koagulieren biologischer Gewebe im Mundbereich mit mindestens einem Betriebsmodus.

Ein bekanntes Problem der Hochfrequenzchirurgie ist die Reproduzierbarkeit und Konstanz der Qualität von Schneide- und/oder Koagulationsvorgängen. Bekanntlich ist ferner die Qualität von Schneide- und/oder Koagulationsvorgängen von der Höhe der hierzu verwendeten Hochfrequenzspannung bzw. von der Intensität der elektrischen Lichtbogen zwischen aktiver Elektrode und Gewebe abhängig (G. Lux und K. Semm "Hochfrequenzdiathermie in der Endoskopie", Springer-Verlag, Berlin und Heidelberg, 1987, Seiten 33ff und 149ff ).

Seit Jahrzehnten werden Hochfrequenz-Chirurgiegeräte benutzt, die auch für zahnärztliche Zwecke Verwendung finden. Die Reproduzierbarkeit der Qualität von Schneide- und/oder Koagulationsvorgängen durch automatisch geregelte Hochfrequenz-Generatoren wurde in den letzten Jahren im Vergleich zu nicht automatisch geregelten Hochfrequenz-Generatoren deutlich verbessert. So sind beispielsweise aus US-A-4 092 986 sowie aus DP-A-0 285 962 Hochfrequenz-Chirurgiegeräte bekannt, deren Ausgangsspannung automatisch konstant geregelt wird. Aus DE-B-25 04 280 sowie EP-A-0 219 568 sind ferner Hochfrequenz-Chirurgiegeräte bekannt, welche mit automatischen Regelkreisen ausgestattet sind, die die Intensität elektrischer Lichtbogen zwischen aktiver Elektrode und Gewebe konstant regeln.

Aus der GB 2 213 381 A ist eine HF-Diathermie-Vorrichtung bekannt, welche Veränderungen der Impedanz bei Behandlung von Gewebe bestimmt, um daraufhin den Hochfrequenz-Strom abzuschalten oder automatisch zu reduzieren. Um eine derartige Impedanzbestimmung vornehmen zu können, wird sowohl die zwischen den Elektroden anliegende Spannung mittels eines Spannungsmessers als auch der fließende Strom mittels eines Strommessers bestimmt. Aus den erhaltenen Meßwerten wird dann eine Impedanzbestimmung vorgenommen und das Überschreiten eines vorgegebenen Impedanzwertes ermittelt. Beim Überschreiten wird entweder ein Alarm ausgelöst oder es erfolgt mittels eines Schalters eine Unterbrechung des HF-Stromkreises zwischen den Elektroden sowie dem zu behandelnden Objekt. Darüber hinaus wird dort vorgeschlagen, eine Konstant-Spannungsquelle vorzusehen, wobei in diesem Falle lediglich der Betrag des fließenden Stromes ermittelt werden muß. In beiden Fällen ist es jedoch notwendig, die Größen Spannung und Strom zu kennen, um eine Impedanzermittlung vorzunehmen. Wenn eine Abschaltung der HF-Diathermie-Vorrichtung nur auf der Basis der Impedanzmessung erfolgt, ist allerdings nicht sichergestellt, daß zumindest örtlich und zeitlich begrenzt solch große Ströme fließen, die zu einer irreversiblen Schädigung von Gewebe führen können.

Insbesondere bei zahnmedizinischen Anwendungen führen aber bereits kurzzeitige Stromerhöhungen zu einer Zerstörung des Pulpagewebes, das sich innerhalb des Zahnes befindet. Diese Gefahr besteht insbesondere dann, wenn der betreffende Zahn mit einer elektrisch leitfähigen Krone versehen ist, die das Zahnfleisch berührt, oder wenn der Zahn mit einer elektrisch leitfähigen Füllung versehen ist, die wiederum zur Pulpa führt.

Gleiches gilt bei Berücksichtigung der in der FR-A-2 417 303 bzw. der DE 29 01 152 A1 offenbarten Sachverhalte, die ein elektrochirurgisches Instrument, umfassend eine Kombination zur Kauterisation und zur Koagulation von Gewebe beschreiben. Im einzelnen ist dort dargelegt, daß zur Vermeidung von Verbrennungen der Strom eines eingesetzten elektrochirurgischen Generators auf maximal 200 mA begrenzt werden muß. Hierfür ist eine entsprechende Schaltung vorgesehen, die zwar bei annähernd konstanten kapazitiven Verhältnissen zwischen der Behandlungselektrode und einer Gegenelektrode eine Leistungsregelung im gewünschten Maße ermöglicht, jedoch resultieren Leckströme im Falle sich plötzlich ändernder Verhältnisse, wie sie beispielsweise dann vorliegen, wenn bei einer zahnmedizinschen Behandlung eine metallische Zahnfüllung berührt wird. Diese Leckströme sind nicht in ausreichendem Maße von der dort vorgesehenen Rückkopplungsschaltung durch Messung eines Spannungsabfalls an einem Widerstand erfaßbar, so daß auch dort die beschriebenen unerwünschten Folgen mangels entsprechender Stromregelung auftreten.

Es ist daher Aufgabe der Erfindung, ein zahnmedizinisches Hochfrequenz-Chirurgiegerät mit einem Hochfrequenzgenerator zum Schneiden und/oder Koagulieren biologischer Gewebe im Mundbereich anzugeben, welches momentan nicht erkennbare Schädigungen von Pulpagewebe oder dergleichen bei ungewollter Berührung mit in der Zahnheilkunde verwendeten metallischen Materialien, die sich im Mundbereich des Patienten befinden, vermeidet.

Die Lösung der Aufgabe der Erfindung erfolgt mit einem Gegenstand gemäß dem Patentanspruch 1, wobei die Unteransprüche mindestens zweckmäßige Ausgestaltungen und Weiterbildungen umfassen.

Der Grundgedanke der Erfindung besteht nun darin, einen den Wirkanteil des Behandlungsstromes erfassenden Wirkstromsensor und eine Stromsteuerungsvorrichtung vorzusehen, welche entweder einen maximal einstellbaren Strompegel begrenzen oder die eine Abschaltung des Hochfrequenzgenerators auslösen, wenn der gemessene Strom den eingestellten maximalen Strompegel erreicht oder übersteigt. Durch die Bestimmung des Wirkanteils des Stromes ist sichergestellt, daß unerwünschte Leckströme nicht fälschlicherweise erfaßt werden, wodurch ein unerwünschtes Abschalten der Vorrichtung mit dem Nachteil einer Beeinträchtigung der Behandlung vermieden werden kann.

Der Erfindung liegt die Erkenntnis zugrunde, daß es selbst bei einer verhältnismäßig kurzzeitigen Berührung beispielsweise einer Zahnfüllung aus Gold vorkommen kann, daß eine Schädigung des Pulpagewebes erfolgt, die weder von dem Zahnarzt noch von dem Patienten bemerkt werden kann. Eine Schädigung von Pulpagewebe bei Berührung einer Goldfüllung oder von sonstigen in der Zahnheilkunde benutzten metallischen Materialien mit der aktiven Elektrode kann deshalb verursacht werden, weil bei allen bisher bekannten Hochfrequenz-Chirurgiegeräten in einem derartigen Fall ein zu großer HF-Strom durch den Wurzelkanal fließen kann.

Mit Hilfe von Vergleichsversuchen konnte an mit Amalgam gefüllten bzw. mit Goldkronen überkronten Schweinegebissen festgestellt werden, daß bei Verwendung von bekannten Hochfrequenz-Chirurgiegeräten deutliche Stromerhöhungen während Schneide- und/oder Koagulationsvorgängen auftraten, sobald die aktive Elektrode eine Amalgamfüllung oder eine Goldkrone berührte, während unter sonst gleichen Bedingungen bei Verwendung eines Hochfrequenz-Chirurgiegeräts gemäß der Erfindung derartige Stromerhöhungen nicht auftraten.

Eine Strombegrenzungs-Einrichtung bei einem Hochfrequenz-Chirurgiegerät gemäß der Erfindung besteht beispielsweise aus einem Strommonitor, welcher ein erstes dem hochfrequenten Strom im Anwendungsteil des Hochfrequenz-Chirurgiegerätes proportionales elektrisches Signal liefert, einem einstellbaren Grenzwertgeber, welcher ein zweites dem jeweils eingestellten maximalen Strompegel proportionales elektrisches Signal liefert und einem Komparator, welcher ein vom Verhältnis des ersten zum zweiten elektrischen Signal abhängiges elektrisches Steuersignal liefert, welches dem Netzteil und/oder dem Hochfrequenz-Generator des Hochfrequenz-Chirurgiegerätes und/oder Warnsignalgebern zugeführt wird, um den hochfrequenten Strom auf den jeweils eingestellten maximalen Pegel zu begrenzen, oder abzuschalten und/oder die Warnsignalgeber zu aktivieren. Der Strommonitor kann beispielsweise in den sekundären oder in den primären Stromkreis des bei Hochfrequenz-Chirurgiegeräten üblichen Ausgangstransformators eingefügt sein.

Bei der Gestaltung des Strommonitors ist hier insbesondere zu beachten, daß bezüglich thermischer Effekte nur der Wirkanteil des hochfrequenten elektrischen Wechselstromes im Anwendungsteil interessiert, so daß beispielsweise ein phasenselektiver Gleichrichter verwendet werden sollte. Da der kapazitive Anteil des hochfrequenten elektrischen Wechselstromes im Anwendungsteil insbesondere durch Streukapazitäten von Bauelementen, Buchsen und Kabeln verursacht wird, kann durch geeignete Gestaltung des Anwendungsteils dieser kapazitive Anteil auf ein Minimum reduziert werden. Dies ist beispielsweise zu erreichen, wenn das Kabel zur aktiven Elektrode elektrisch abgeschirmt wird, diese Schirmung innerhalb des Hochfrequenz-Chirurgiegerätes zusammen mit der Leitung zur Neutralelektrode auf Erdpotential geschaltet wird, wie es weiter unten in einem Ausführungsbeispiel dargestellt und beschrieben ist.

Außerdem sollte beachtet werden, daß insbesondere bei amplitudenmodulierten Strömen das elektrische Ausgangssignal des Strommonitors einem Strom-Zeit-Integral entspricht, welches der Wärmekapazität des thermisch gefährdeten Gewebevolumens angepaßt ist, wobei vorzugsweise der Effektivwert bzw. der quadratische Mittelwert des mehr oder weniger von der Sinusform abweichenden und mehr oder weniger amplitudenmodulierten hochfrequenten Wechselstromes zu berücksichtigen ist.

In einer weiteren Ausgestaltung der Erfindung wird das erfindungsgemäße Hochfrequenz-Chirurgiegerät zusätzlich mit einer Anzeigevorrichtung ausgestattet, auf welcher der jeweils eingestellte maximale Strompegel angezeigt wird.

Ist ein Hochfrequenz-Chirurgiegerät mit mehreren Betriebsmodi ausgestattet, z.B. den Schneide-Modi koagulationsfreier Schnitt, koagulierender Schnitt und/oder den Koagulations-Modi Soft-Koagulation, Forced-Koagulation oder Spray-Koagulation, so kann jedem Betriebs-Modus eine separate Strombegrenzungs-Einrichtung und/oder ein separat einstellbarer, maximaler Strompegel zugeordnet werden.

Anhand der Zeichnung soll die Erfindung beispielsweise näher erläutert werden.
Es zeigen:

| | |
|---|---|
| Fig. 1 | einen schematischen Teilschnitt durch ein Gebiß, |
| Fig. 2 | eine Draufsicht auf ein Gebiß, wobei ein Eingriff mit einer aktiven Elektrode dargestellt ist, |
| Fig. 3 bis Fig. 6 | Blockschaltbilder unterschiedlicher Ausführungsbeispiele eines Hochfrequenz-Chirurgiegerätes gemäß der Erfindung, |
| Fig. 7 | eine Ansicht einer Frontplatte eines Hochfrequenz-Chirurgiegerätes gemäß der Erfindung; und |
| Fig. 8 | eine Teildarstellung eines Blockschaltbildes eines im Vergleich zu den Ausführungsbeispielen in Fig. 3 bis 6 abgewandelten Ausführungsbeispiels. |

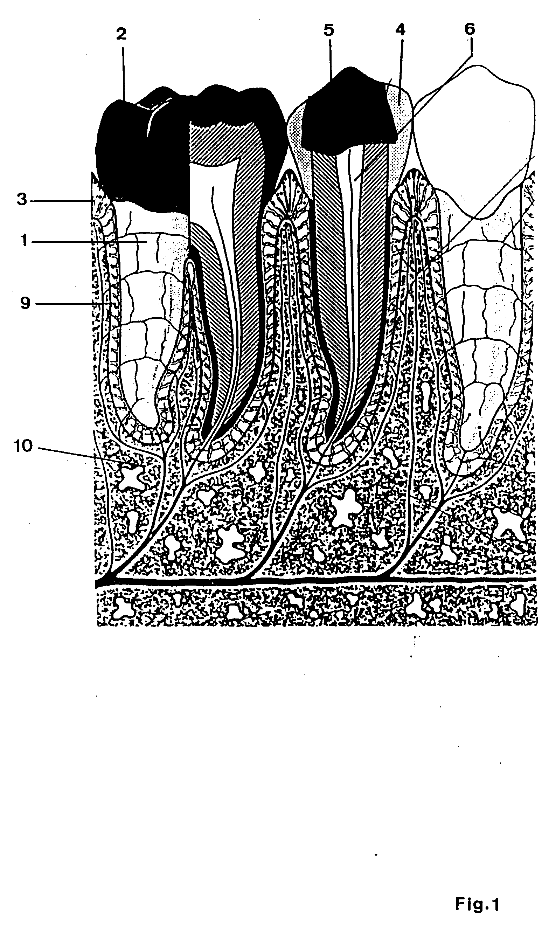

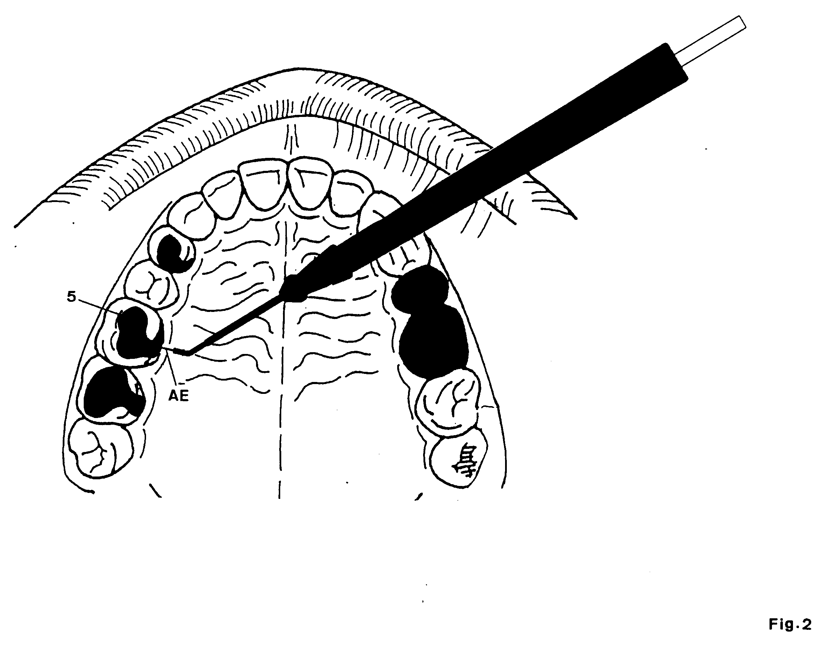

In Figur 1 ist schematisch ein Ausschnitt eines Gebissesdargestellt, wobei der Zahn 1 mit beispielsweise einer elektrisch leitfähigen Krone 2, überkront ist, welche Zahnfleisch 3 berührt, und der Zahn 4 mit einer elektrisch leitfähigen Füllung 5, beispielsweise einer Amalgamfüllung, gefüllt ist, welche die Pulpa 6 berührt. Berührt die aktive Elektrod AE eines Hochfrequenz-Chirurgiegerätes, wie in Figur 2 schematisch dargestellt, eine Amalgamfüllung 5, so kann elektrischer Strom durch die Amalgamfüllung in die Pulpa fließen, wodurch das Pulpagewebe innerhalb kurzer Zeit thermisch zerstört werden kann. Das Pulpagewebe ist für die Vitalität und das Reaktionsvermögen eines Zahnes wichtig. Der operierende Zahnarzt bemerkt diese Komplikation nicht, weil das Pulpagewebe sich innerhalb des Zahnes befindet.

Berührt die aktive Elektrode AE eines Hochfrequenz-Chirurgiegerates wie in Fig. 2 dargestellt eine Goldkrone, welche in der Regel mit Zahnfleisch elektrisch leitfähig in Kontakt ist, so kann elektrischer Strom durch die Goldkrone in das angrenzende Zahnfleisch fließen und dieses innerhalb kurzer Zeit thermisch zerstören. Es kann angenommen werden, daß der elektrische Strom hierbei bevorzugt durch das Desmodont 9 des betreffenden Zahnes fließt und dieses thermisch zerstört. Das Desmodont ist ein faserreiches, derbes Bindegewebe, welches die Zahnwurzel im Kieferknochen hält. Es ist der Halteapparat eines Zahnes. Der operierende Zahnarzt bemerkt diese Komplikation nicht, weil das Desmodont nicht sichtbar im relativ engen Spalt zwischen Zahnwurzel und Kieferknochen 10 angeordnet ist.

Messungen der Stromstärke während der Anwendung der Hochfrequenzchirurgie an mit Amalgam gefüllten bzw. mit Goldkronen überkronten Schweinegebissen ergaben deutliche Stromerhöhungen während Schneide- und/oder Koagulationsvorgängen sobald die aktive Elektrode AE Amalgamfüllungen bzw. Goldkronen berührt. Gleiche Komplikationen können in der Zahnheilkunde entstehen, wenn die aktive Elektrode elektrisch leitfähige Spangen, Instrumente, Implantate etc. berührt. Ähnliche Komplikationen können auch in anderen chirurgischen Fachbereichen entstehen und zwar immer dann, wenn eine aktive Elektrode elektrisch gut leitfähige Teile berührt, deren elektrisch leitfähige Kontaktfläche mit Gewebe des Patienten größer ist als die beabsichtigte Kontaktfläche zwischen aktiver Elektrode und Gewebe des Patienten.

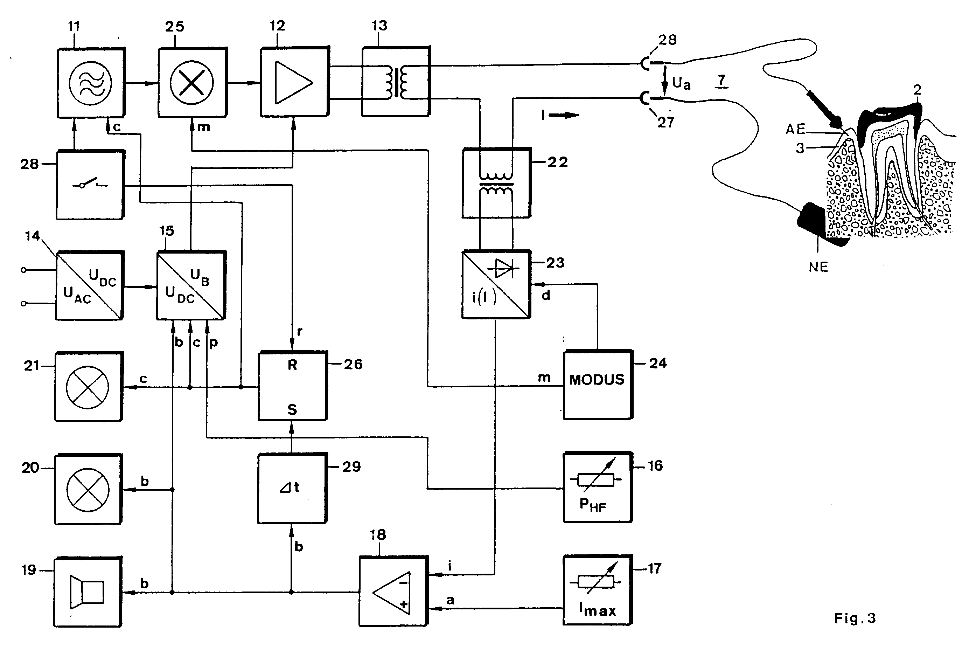

In Figur 3 ist in Form eines Blockschaltbildes das Prinzip eines erfindungsgemäßen Hochfrequenz-Chirurgiegerätes dargestellt. Der Hochfrequenz-Generator dieses Ausführungsbeispieles entspricht prinzipiell denen bekannter Hochfrequenz-Chirurgiegeräte. Er besteht aus den an sich bekannten Funktionselementen Hochfrequenz-Oszillator 11, Amplitudenmodulator 25, Hochfrequenz-Leistungsverstärker 12, Ausgangstransformator 13, Netzteil 14, 15 und Einstellvorrichtung 16 für die Hochfrequenz-Ausgangsleistung $P_{HF}$ Die Einstellung der Hochfrequenz-Ausgangsleistung $P_{HF}$ erfolgt bei bekannten Hochfrequenz-Chirurgiegeräten der oben beschriebenen Art beispielsweise durch Variation der Betriebsspannung $U_B$ mittels eines geeigneten Stellmittels 16. Der hochfrequente Strom I im Anwendungsteil 7 eines derartigen Hochfrequenz-Chirurgiegerätes ist abhängig von der Spannung $U_a$ am Ausgang 27, 28 des Gerätes und dem elektrischen Widerstand des Lastkreises. Der elektrische Widerstand des Lastkreises wird während Schneide- und/oder Koagulationsvorgängen hauptsächlich durch den elektrischen Übergangswiderstand zwischen aktiver Elektrode AE und biologischem Gewebe 3 bestimmt, wobei dieser Übergangswiderstand infolge der relativ kleinen Kontaktfläche zwischen aktiver Elektrode und Gewebe relativ groß ist.

Berührt die relativ kleinflächige aktive Elektrode AE statt des zu schneidenden und/oder zu koagulierenden Gewebes 3 elektrisch leitfähige Teile, wie beispielsweise Goldkronen 2, Amalgamfüllungen 5 oder metallische Instrumente, so kann infolge der in der Regel relativ großen Kontaktfläche dieser elektrisch leitfähigen Teile zum Gewebe des Patienten ein relativ kleiner elektrischer Widerstand und damit relativ zum beabsichtigten Schneide- und/oder Koagulationsvorgang ein großer Strom I entstehen, der unbeabsichtigt Gewebe thermisch zerstört.

Deswegen ist das Hochfrequenz-Chirurgiegerät entsprechend Figur 3 zusätzlich mit einer Strombegrenzungs-Einrichtung 22, 23, 17, 18 ausgestattet. Eine derartige Strombegrenzungs-Einrichtung besteht beispielsweise aus einem Strom-Sensor 22, 23, welcher ein dem Strom I im Anwendungsteil proportionales elektrisches Signal $i = f(I)$ bildet. Dieses Signal wird einem Komparator 18 zugeführt, welcher ein Steuersignal b an das Netzteil 15 liefert, wenn das Signal $i = f(I)$ gleich oder größer wird als ein Referenz-Signal $a = f(I_{max})$, wobei $I_{max}$ der an der Einstelleinrichtung 17 vom Anwender einstellbare Maximalwert des Stromes I ist.

Das Signal b kann das Netzteil 15 entweder so steuern, daß I den Maximalwert $I_{max}$ nicht übersteigt oder die Betriebsspannung $U_B$ und/oder den Oszillator 11 sofort oder verzögert abschaltet, wenn I gleich oder größer $I_{max}$ wird. Das Signal b kann zusätzlich akustischen 19 und/oder optischen 20, 21 Signalgebern zugeführt werden, welche Signale generieren, wenn I gleich oder größer $I_{max}$ ist.

Die Verzögerung $\Delta t$ des Signals b durch das Verzögerungsglied 29 ist dann zweckmäßig, wenn der Strom I kurzzeitig größer sein soll als $I_{max}$ und/oder die Betriebsspannung $U_B$ und/oder der Oszillator 11 nicht sofort sondern um $\Delta t$ verzögert abschalten soll. Dies ist beispielsweise beim Anschneidevorgang vorteilhaft, wenn der Strom I eingeschaltet wird während die aktive Elektrode AE das Gewebe 3 bereits berührt, denn in dieser Situation ist bekanntlich kurzzeitig ein größerer Strom erforderlich als während Schneidevorgängen.

Soll das Signal b die Betriebsspannung $U_B$ und/oder den Oszillator 11 sofort oder verzögert abschalten, so ist zusätzlich zum Speicherelement 26, beispielsweise ein RS-Flip-Flop erforderlich, welches ein elektrisches Signal c liefert, wenn das Signal b am Setzeingang S des Speicherelementes 26 erscheint. Das Signal c wird dem Netzteil 15 und/oder dem Oszillator 11 zugeführt, um die Betriebsspannung $U_B$ und/oder den Oszillator 11 abzuschalten. Das Signal c bleibt auch nach dem Verschwinden des Signals b solange erhalten, bis durch erneutes Betätigen eines Schalters 28, beispielsweise des zum Aktivieren des Oszillators in der Regel vorhandenen Finger- oder Fußschalters, ein elektrisches Signal r an den Rücksetzeingang R des Speicherelementes 26 geliefert wird, welches das Signal c löscht.

Das Signal c kann zusätzlich einem optischen und/oder akustischen Signalgeber 21 zugeleitet werden.

Soll das Hochfrequenz-Chirurgiegerät verschiedene Betriebsmodi haben, z.B. daß die Ausgangsspannung mehr oder weniger in der Amplitude moduliert ist, so ist es in weiterer Ausgestaltung der Erfindung zweckmäßig, dem Demodulator 23 aus der Modus-Einstellvorrichtung 24 ein elektrischen Signal d zuzuleiten, welches beispielsweise die Integrationszeitkonstante des Demodulators der jeweils gewählten Modulation anpaßt, so daß das Signal i beispielsweise dem Effektivwert des Stromes I auch dann proportional ist, wenn die Ausgangsspannung mehr oder weniger stark in der Amplitude moduliert ist.

Die Modulation der Ausgangsspannung $U_a$ erfolgt durch das Signal m, welches die Modus-Einstellvorrichtung 24 generiert und welches beispielsweise dem Amplitudenmodulator 25 zugeführt wird.

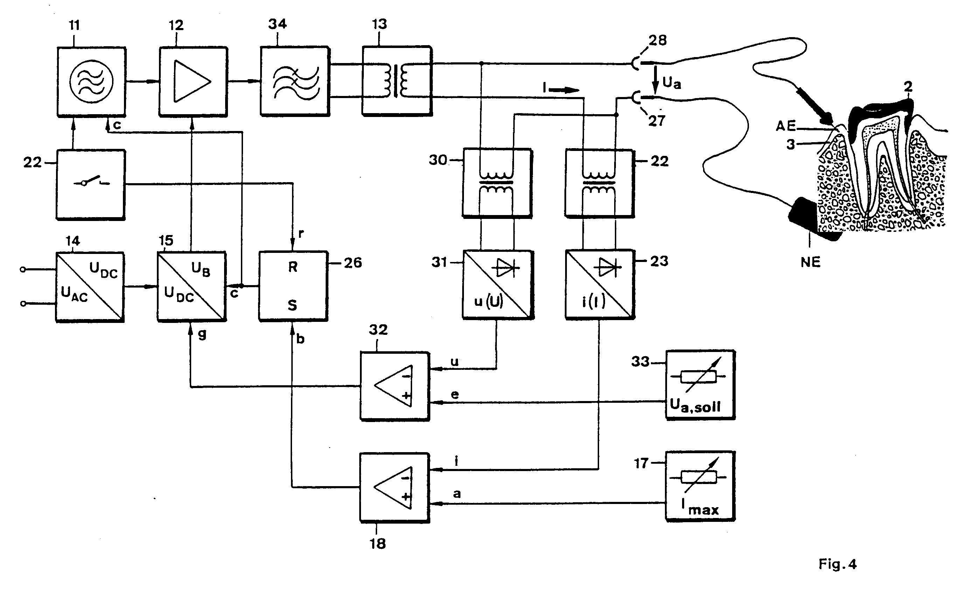

In Figur 4 ist in Form eines Blockschaltbildes ein weiteres Ausführungsbeispiel eines erfindungsgemäßen Hochfrequenz-Chirurgiegerätes dargestellt. Der Hochfrequenz-Generator dieses Ausführungsbeispieles entspricht prinzipiell denen bekannter Hochfrequenz-Chirurgiegeräte mit automatischer Regelung der HF-Ausgangsspannung, beispielsweise EP-A-0285 962. Er besteht aus den an sich bekannten, auch in Fig. 3 dargestellten Funktionselementen Hochfrequenz-Oszillator 11, Hochfrequenz-Leistungsverstärker 12, Ausgangstransformator 13, Netzteil 14, 15 und, abweichend von Fig. 3, einem Sollwertgeber 33 für die HF-Ausgangsspannung $U_{a\ soll}$ am Ausgang 27, 28 des Hochfrequenz-Chirurgiegerätes. Die automatische Regelung der HF-Ausgangsspannung $U_a$ erfolgt in an sich bekannter Weise durch einen HF-Spannungssensor 30, 31, der ein der HF-Spannung $U_a$ proportionales elektrisches Signal $u = f(U_a)$ liefert, welches einem Komparator 32 zugeführt wird, wo es mit einem Sollwertsignal e des Sollwertgebers 33 verglichen wird. Das Ausgangssignal g des Komparators 32 wird in geeigneter Weise dem Netzteil 15 zugeführt, um die Betriebsspannung $U_B$ des Leistungsverstärkers 12 so zu steuern, daß die HF-Ausgangsspannung $U_a$ dem am Sollwertgeber 33 eingestellten Wert entspricht. Für die automatische Regelung der HF-Ausgangsspannung $U_a$ ist es zweckmäßig, daß der Hochfrequenz-Generator möglichst oberwellenarme Sinusspannung liefert, weswegen zwischen HF-Leistungsverstärker 12 und Ausgangstransformator 13 ein Tiefpaßfilter 34 geschaltet ist.

Die Strombegrenzungs-Einrichtung und alle weiteren Funktionen dieses Ausführungsbeispieles entsprechen prinzipiell dem in Fig. 3 dargestellten und dort beschriebenen Ausführungsbeispiels. Da Hochfrequenz-Chirurgiegeräte mit automatischer Regelung der HF-Ausgangsspannung $U_a$ in der Regel keine Amplitudenmodulation der HF-Ausgangsspannung $U_a$ haben, wurden die hierfür in Fig. 3 vorgesehenen Elemente 24 und 25 in Fig. 4 weggelassen.

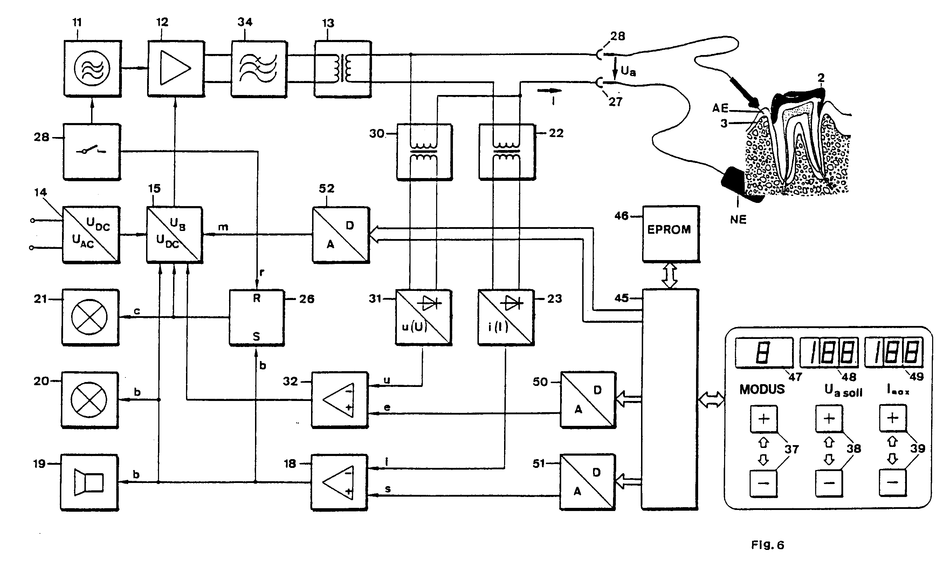

Ein Ausführungsbeispiel eines Hochfrequenz-Chirurgiegerätes mit automatischer Regelung der HF-Ausgangsspannung $U_a$, deren Amplitude beispielsweise pulsierend moduliert ist, wird anhand von Fig. 6 beschrieben.

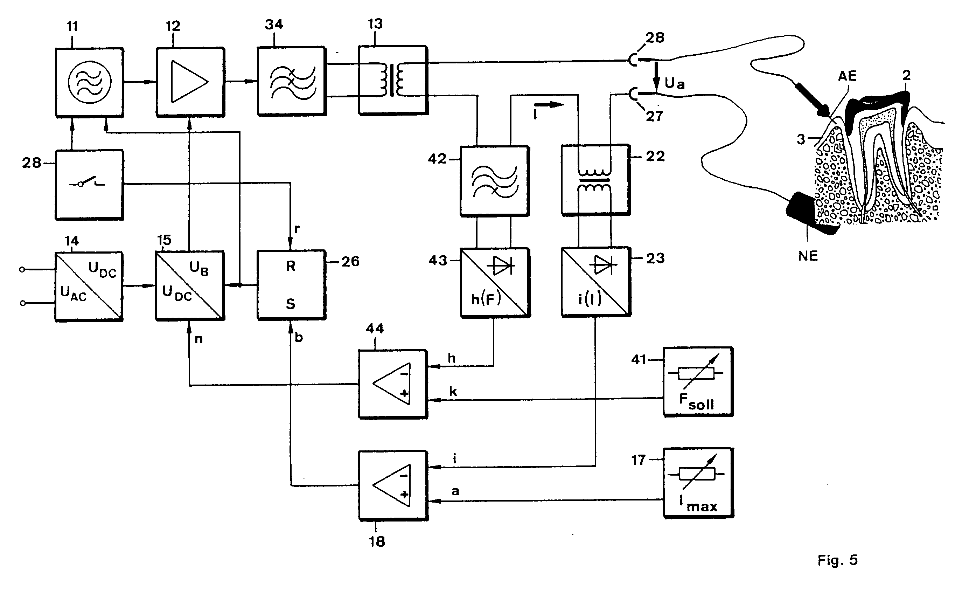

In Figur 5 ist in Form eines Blockschaltbildes ein weiteres Ausführungsbeispiel eines erfindungsgemäßen Hochfrequenz-Chirurgiegerätes dargestellt. Der Hochfrequenz-Generator dieses Ausführungsbeispieles entspricht prinzipiell denen bekannter Hochfrequenz-Chirurgiegeräte mit automatischer Regelung der Intensität der elektrischen Lichtbogen zwischen aktiver Elektrode und Gewebe, beispielsweise EP 0219 568. Er besteht aus den an sich bekannten, auch in Fig. 3 dargestellten Funktionselementen Hochfrequenz-Oszillator 11, Hochfrequenz-Leistungsverstärker 12, Ausgangstransformator 13, Netzteil 14, 15 und, abweichend von Fig. 3, einem Sollwertgeber 41, an welchem der Sollwert der Intensität der elektrischen Lichtbogen eingestellt werden kann.

Die automatische Regelung der elektrischen Lichtbogen erfolgt in an sich bekannter Weise durch einen Lichtbogen-Sensor, bestehend aus einem Frequenzfilter 42 und einem Gleichrichter 43, welcher ein der Intensität der elektrischen Lichtbogen zwischen aktiver Elektrode AE und Gewebe 3 des Patienten proportionales elektrisches Signal h liefert, welches einem Komparator 44 zugeführt wird, wo es mit einem Sollwert-Signal k des Sollwertgebers 41 verglichen wird. Das Ausgangssignal n des Komparators 44 wird in geeigneter Weise dem Netzteil 15 zugeführt, um die Betriebsspannung $U_B$ so zu steuern, daß die Intensität F der elektrischen Lichtbogen der am Sollwertgeber 41 eingestellten Intensität $F_{soll}$ entspricht. Hierbei ist es zweckmäßig, daß der Hochfrequenz-Generator oberwellenfreie, sinusförmige HF-Spannung an den Ausgang 27, 28 liefert, weswegen ein Tiefpaßfilter 34 zwischen HF-Leistungsverstärker 12 und Ausgangstransformator 13 eingefügt ist.

Die Strombegrenzungs-Einrichtung und alle weiteren Funktionen dieses Ausführungsbeispieles entsprechen prinzipiell dem in Fig. 4 dargestellten und dort beschriebenen Ausführungsbeispiel.

Da Hochfrequenz-Chirurgiegeräte mit automatischer Regelung der Intensität F der elektrischen Lichtbogen zwischen aktiver Elektrode AE und Gewebe 3 in der Regel keine Amplitudenmodulation der HF-Ausgangsspannung $U_a$ haben, wurden die hierfür in Fig. 3 vorgesehenen Elemente 24 und 25 in Fig. 4 weggelassen.

Ein Ausführungsbeispiel eines Hochfrequenz-Chirurgiegerätes mit automatischer Regelung der Intensität F der elektrischen Lichtbogen zwischen aktiver Elektrode AE und Gewebe 3 ist anhand von Fig. 6 beschrieben.

Anhand von Fig. 6 wird im folgenden eine weitere Ausgestaltung der in den Fig. 3, 4 und 5 dargestellten Ausführungsbeispiele beschrieben.

Bei Anwendung eines Mikrokontrollers oder Mikroprozessors 45 kann die Einstellung der Parameter Modus, HF-Ausgangsleistung $P_{HFsoll}$ oder HF-Ausgangsspannung $U_{HFsoll}$ oder Intensität $F_{soll}$ der elektrischen Lichtbogen zwischen aktiver Elektrode AE und Gewebe 3, sowie Maximalwert $I_{max}$ des Stromes I digital, beispielsweise über AUF-ABWÄRTS-Tasten 37, 38, 39 erfolgen.

Außerdem können die jeweils eingestellten Parameter beispielsweise auf alphanumerischen Displays 47, 48, 49 angezeigt werden.

Die Anwendung eines Mikrokontrollers oder Mikroprozessors 45 in einem erfindungsgemäßen Hochfrequenz-Chirurgiegerät hat auch den Vorteil, daß bei Anwendung von Modi, welche eine Amplitudenmodulation der HF-Ausgangsspannung $U_a$ erfordern, die zum jeweils gewählten Modus relevanten Amplitudenmodulationsart aus einem Speicher 46, beispielsweise einem EPROM, automatisch abgerufen werden und über einen Digital/Analog-Wandler 52 entweder einem Amplitudenmodulator 25, wie in Fig. 3 dargestellt, oder dem Netzteil 15 zugeführt werden kann. Das elektrische Signal n ist hierbei das Modulationssignal.

Die Anwendung eines Mikrokontrollers oder Mikroprozessors 45 in einem erfindungsgemäßen Hochfrequenz-Chirurgiegerät hat insbesondere den Vorteil, daß bei Modi, welche eine Amplitudenmodulation der HF-Ausgangsspannung $U_a$ enthalten, die Ermittlung der Effektivwerte für das elektrische Signal i = f (I) nicht per Hardware, beispielsweise mittels relativ teurer Effektivwertgleichrichter, sondern mittels Software durch den Mikroprozessor 45 z.B. in der Weise erfolgen kann, daß das elektrische Signal a = f ($I_{max}$) mit einem geeigneten Formfaktor, beispielsweise dem sog. Crestfaktor C, multipliziert wird. Mit Crestfaktor ist hier das mathematische Verhältnis von Spitzenwert zu Effektivwert beispielsweise der HF-Ausgangsspannung $U_a$ gemeint, wobei die Integrationszeitkonstante der Periodendauer der Amplitudenmodulation oder einem ganzzahligen Vielfachen hiervon entsprechen muß.

So kann der Mikroprozessor 45 für jeden Modus einen passenden Formfaktor liefern, der einem Digital/Analogwandler 51 zugeführt wird, welcher ein elektrisches Signal s liefert, welches dem Produkt aus Spitzenwert von a = f ($I_{max}$) und dem jeweiligen Crestfaktor C von $U_a$ entspricht.

Je nach gewähltem Modus und/oder gewählter HF-Ausgangsspannung $U_{a\ soll}$ bzw. gewählter Intensität $F_{soll}$ der elektrischen Lichtbogen zwischen aktiver Elektrode AE und Gewebe 3 kann der Mikroprozessor auch das jeweils per Software definierte elektrische Signal e bzw. k generieren, wofür bekanntlich noch ein Digital/Analogwandler 50 erforderlich ist.

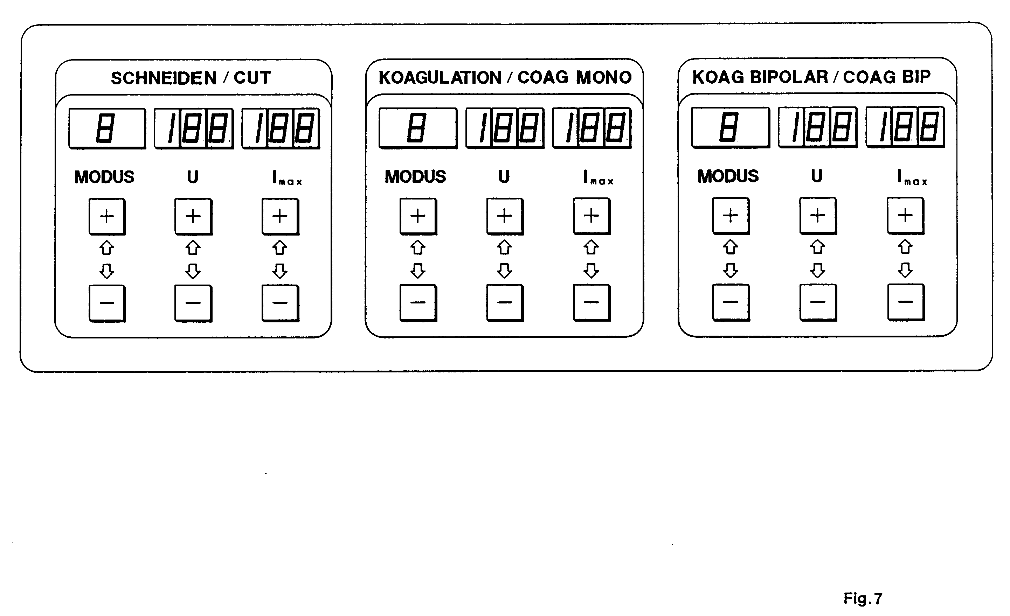

In Figur 7 ist ein Ausführungsbeispiel einer Frontplatte eines erfindungsgemäßen Hochfrequenz-Chirurgiegerätes dargestellt. Sie ist beispielsweise mit drei Funktionsfeldern, u.zw. Schneiden, Koagulation monopolar und Koagulation bipolar ausgestattet. In jedem Funktionsfeld sind beispielsweise zwei bei bekannten Hochfrequenz-Chirurgiegeräten übliche Einstellvorrichtungen, nämlich für Modus und Ausgangsspannung U vorhanden. Modus meint bei Schneiden beispielsweise den Koagulationsgrad oder Schnittränder bzw. koagulationsarmer und koagulierender Schnitt, bei Koagulation monopolar beispielsweise Soft-Koagulation, Forced-Koagulation oder Spray-Koagulation, bei Koagulation bipolar beispielsweise Mikro- oder Makro-Koagulation. U meint beispielsweise die HF-Ausgangsspannung, automatisch geregelt oder nicht, oder die Intensität der elektrischen Lichtbogen zwischen aktiver Elektrode und Gewebe, oder die HF-Ausgangsleistung in Watt.

Zusätzlich ist eines oder mehrere Funktionsfelder erfindungsgemäß mit je einer Einstellvorrichtung für die Strombegrenzung ausgestattet, an welcher der maximale Pegel $I_{max}$ des hochfrequenten Wechselstromes im Anwendungsteil eingestellt werden kann.

Zur Vermeidung kapazitiver Ströme zwischen den Leitungen 55 und 56 sowie zwischen den Kabeln 53 und 54 ist sowohl die Leitung 55 wie auch das Kabel 54 elektrisch geschirmt. Die Schirmung 58 der Leitung 55 und des Kabels 54, die in der Ausgangsbuchse 28 elektrisch leitfähig miteinander verbunden sind, muß so an die Leitung 56 elektrisch angeschlossen sein, daß der kapazitive Strom $I_c$ nicht durch den HF-Strom-Sensor 22,23 fließt. Der HF-Strom-Sensor 22,23 ist deswegen zwischen der Anschlußstelle 57 der Schirmung 58 an die Leitung 56 und der Ausgangsbuchse 27 eingefügt.

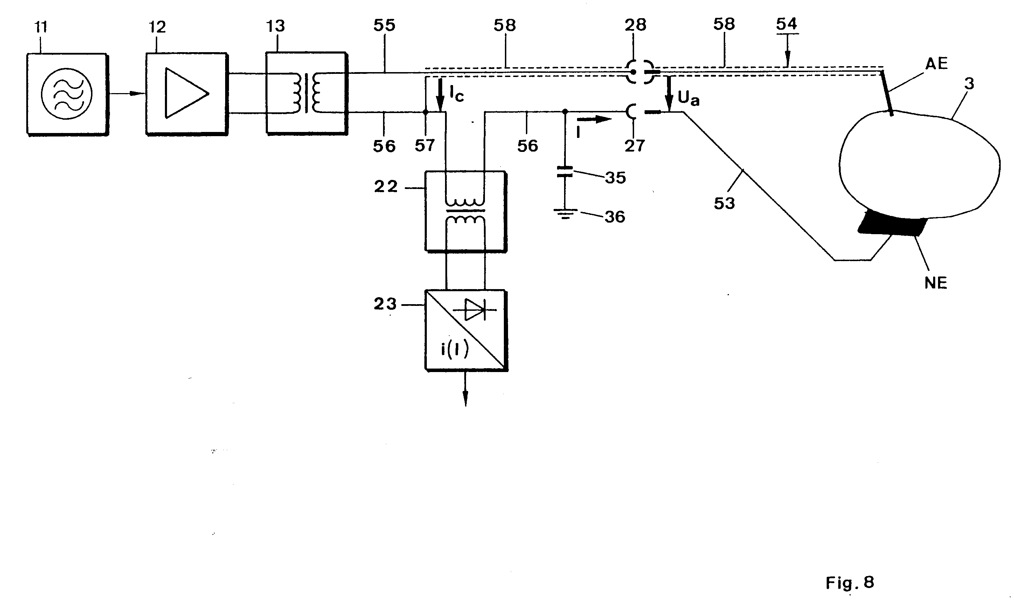

Diese Lösung ist sowohl für Hochfrequenz-Chirurgiegeräte mit sog. floating output als auch bei Hochfrequenz-Chirurgiegeräten mit direkter oder kapazitiver Erdung der Neutralelektrode anwendbar. In Fig. 8 ist schematisch ein Hochfrequenz-Chirurgiegerät mit kapazitiver Erdung dargestellt, wobei ein Kondensator 35 zwischen die Leitung 56 und dem Schutzleiterpotential 36 des Hochfrequenz-Chirurgiegerätes geschaltet ist.

Bei Geräten mit sog. floating output fehlt der Kondensator 35. Bei Geräten mit direkter Erdung der Neutralelektrode ist statt des Kondensators 35 eine niederohmige, konduktive Verbindung vorhanden.

## Patentansprüche

1. Zahnmedizinisches Hochfrequenz-Chirurgiegerät mit einem Hochfrequenzgenerator zum Schneiden und/oder Koagulieren biologischer Gewebe im Mundbereich mit mindestens einem Betriebsmodus sowie mit einer Einstelleinrichtung zur Einstellung mindestens eines HF-Ausgangsparameters, wobei mindestens ein den Wirkanteil des Behandlungsstroms erfassender Wirkstromsensor (22, 23) und eine Stromsteuerungseinrichtung (17, 18) vorgesehen ist, an der entweder ein maximaler Wirk-Strompegel ($I_{max}$) von außen am Gerät einstellbar ist und mit deren Hilfe der HF-Strompegel auf einen für den betreffenden Betriebsmodus eingestellten maximalen Wirk-Strompegel begrenzt wird oder wobei die Stromsteuerungseinrichtung (17, 18) den Hochfrequenzgenerator (11) dann abschaltet, wenn der Behandlungsstrom den eingestellten, maximalen Wirk-Strompegel erreicht oder übersteigt.

2. Kochfrequenz-Chirurgiegerät nach Anspruch (1), **gekennzeichnet durch**
eine Warnsignaleinrichtung (19), die dann aktiviert wird, wenn der eingestellte, maximale Wirk-Strompegel erreicht oder überstiegen wird.

3. Hochfrequenz-Chirurgiegerät nach Anspruch 1, **dadurch gekennzeichnet,**
daß der Wirkstromsensor einen phasenselektiven Gleichrichter (23) aufweist.

**Claims**

1. A dental high-frequency surgical instrument with a high-frequency generator for the cutting and/or coagulating of biological tissue in the region of the mouth with at least one operating mode and with an adjusting device for adjusting at least one HF initial parameter, in which at least one energy current sensor (22, 23) is provided, detecting the effective component of the treatment current, and a current control device (17, 18) is provided, at which either a maximum energy current level ($I_{max}$) is adjustable from the exterior on the instrument and with the aid of which the HF current level is limited to a maximum energy current level set for the operating mode concerned, or in which the current control device (17, 18) switches off the high-frequency generator (11) when the treatment current reaches or exceeds the set, maximum energy current level.

2. A high-frequency surgical instrument according to Claim 1,
characterised by
a warning signal device (19) which is activated when the set maximum energy current level is reached or exceeded.

3. A high-frequency surgical instrument according to Claim 1,
characterised in that
the energy current sensor has a phase-selective rectifier (23).

**Revendications**

1. Appareil de dentisterie pour électro-chirurgie à haute fréquence comprenant un générateur haute fréquence pour couper et/ou coaguler des tissus biologiques dans l'espace buccal comportant au moins un mode de fonctionnement de même qu'un dispositif de réglage pour ajuster au moins un paramètre de sortie-HF, dans lequel au moins un détecteur de courant actif (22, 23) détecte la composante active du courant de traitement et un dispositif de commande de courant (17, 18) est prévu, par lequel, soit un niveau de courant actif maximal ($I_{max}$) est ajustable de manière externe et à l'aide duquel le niveau du courant-HF peut être limité à un niveau de courant actif maximal ajusté pour le mode de fonctionnement correspondant, soit le dispositif de commande de courant (17, 18) coupe le générateur haute fréquence (11) lorsque le courant de traitement atteint ou dépasse le courant actif maximal ajusté.

2. Appareil d'électro-chirurgie à haute fréquence selon la revendication 1, caractérisé en ce qu'un dispositif de signal d'avertissement (19) est activé lorsque le niveau de courant actif maximal ajusté est atteint ou dépassé.

3. Appareil pour électro-chirurgie à haute fréquence selon la revendication 1, caractérisé en ce que le détecteur de courant actif comporte un redresseur de phase sélectif (23).

**Fig.1**

Fig . 2

Fig. 3

EP 0 495 140 B1

Fig. 4

EP 0 495 140 B1

Fig. 5

Fig. 6

Fig.7

Fig. 8

EP 0 495 140 B1